(19) Europäisches Patentamt
European Patent Office
Office européen des brevets

(11) **EP 2 349 681 B1**

(12) **EUROPÄISCHE PATENTSCHRIFT**

(45) Veröffentlichungstag und Bekanntmachung des Hinweises auf die Patenterteilung:
**24.09.2014 Patentblatt 2014/39**

(21) Anmeldenummer: **09767994.8**

(22) Anmeldetag: **30.10.2009**

(51) Int Cl.:
**B29C 61/06** (2006.01)     **A61B 17/00** (2006.01)

(86) Internationale Anmeldenummer:
**PCT/EP2009/064367**

(87) Internationale Veröffentlichungsnummer:
**WO 2010/049521 (06.05.2010 Gazette 2010/18)**

(54) **ARTIKEL MIT MINDESTENS EINEM THERMO-PROGRAMMIERBAREN SCHALTABSCHNITT FÜR ANWENDUNGEN IN KONTAKT MIT MATERIALIEN MIT HOHEN WÄRMEÜBERGANGSZAHLEN**

ITEM WITH AT LEAST ONE THERMOPROGRAMMABLE SWITCHING SECTION FOR APPLICATIONS IN CONTACT WITH MATERIALS HAVING HIGH HEAT TRANSFER COEFFICIENTS

ARTICLE AYANT AU MOINS UNE PARTIE DE COMMUTATION THERMOPROGRAMMABLE POUR DES APPLICATIONS EN CONTACT AVEC DES MATÉRIAUX PRÉSENTANT DES COEFFICIENTS DE TRANSFERT THERMIQUE ÉLEVÉS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **31.10.2008 DE 102008043357**
**05.03.2009 DE 102009001356**

(43) Veröffentlichungstag der Anmeldung:
**03.08.2011 Patentblatt 2011/31**

(73) Patentinhaber: **Helmholtz-Zentrum Geesthacht Zentrum für Material- und Küstenforschung GmbH**
**21502 Geesthacht (DE)**

(72) Erfinder:
• **WEIGEL, Thomas**
**14552 Michendorf (DE)**
• **LENDLEIN, Andreas**
**14167 Berlin (DE)**
• **LÜTZOW, Karola**
**14195 Berlin (DE)**
• **KLEIN, Frank**
**14089 Berlin (DE)**
• **MADBOULY, Samy**
**EG/12613 Cairo (DE)**

(74) Vertreter: **Gulde & Partner Patent- und Rechtsanwaltskanzlei mbB Wallstraße 58/59 10179 Berlin (DE)**

(56) Entgegenhaltungen:
**US-A1- 2005 212 630    US-A1- 2007 173 800**
**US-A1- 2008 004 692**

**Beschreibung**

Formgedächtniskunststoffe können neben einer sichtbaren temporären Form noch eine permanente ursprüngliche Gestalt gespeichert haben. Durch Auslösung eines äußeren Stimulus wie beispielsweise eine Temperaturerhöhung kann die permanente Form nahezu vollständig wieder abgerufen werden, d.h. der Kunststoff "erinnert sich" an seine ursprüngliche Gestalt.

Das Material wird dafür zunächst mittels konventioneller Verarbeitungsmethoden, wie Extrusion, Spritzguss oder auch aus der Polymerlösung in seine ursprüngliche, permanente Form gebracht. Anschließend wird der Kunststoff deformiert und in der gewünschten temporären Form fixiert. Dieser Vorgang wird als Programmierung bezeichnet. Er besteht entweder aus einem Erwärmen der Probe oberhalb der Schalttemperatur $T_{sw}$ und unterhalb der höchsten Übergangstemperatur $T_{perm}$, einer Deformation unter Ausübung eines Formenzwangs und einem Abkühlvorgang unter Aufrechterhaltung des Formenzwangs. Alternativ kann die Programmierung durch Deformation bei niedriger Temperatur unterhalb der Schalttemperatur $T_{sw}$ erfolgen ("kaltes Verstrecken").

Die permanente Form ist nun gespeichert, während die temporäre Form aktuell vorliegt. Durch Erwärmen des Kunststoffes auf eine Temperatur höher als die Schalttemperatur Tsw kommt es zum Auslösen des Formgedächtniseffekts, und das Material geht aufgrund seiner Entropieelastizität in die gespeicherte permanente Form zurück.

Erfolgt der Übergang von temporärer zu permanenter Form auf Grund einer Temperaturänderung, handelt es sich um einen thermisch induzierten Formgedächtniseffekt. Dieser thermisch induzierte Formgedächtniseffekt wird in den meisten Fällen durch direkte Temperatureinwirkung (Wärmezufuhr) realisiert. Darüber hinaus ist auch eine indirekte Aufheizung bekannt. Beispielsweise ist bekannt, dass nanoskalige Partikel in einen Formgedächtniskunststoff eingearbeitet werden können, welche die Fähigkeit besitzen, mit einem extern erzeugten alternierenden elektromagnetischen Feld (EMF) in Wechselwirkung zu treten. Auf diese Weise kann "indirekt" über die eingebetteten Nanopartikel ein Temperaturanstieg im Formgedächtnispolymer erreicht werden.

Die Fähigkeit solcher Nanopartikel, durch Anregung über ein EMF eine Erwärmung zu realisieren, wurde bereits bei verschiedenen Anwendungen verwirklicht. Ausgenutzt wurden hierbei die, bei leitfähigen Partikeln im EMF induzierten, elektrischen Wirbelströme die eine Wärmeentwicklung zur Folge haben. Dieses Prinzip fand nicht nur Eingang in technologische Anwendungen, z. B. als innovative Klebetechnik zur Aushärtung von Klebern (WO 2004/056156 A, WO 2002/012409 A), sondern auch bei biomedizinischen Anwendungen wurden schon Nanopartikel etwa zur Abtötung von Metastasen von Tumoren mittels kurzzeitiger lokaler Überhitzung des Körpergewebes angewandt (US 2004219130 A).

Die Verwendung von Zusatzstoffen zu Formgedächtnispolymeren war bisher zumeist Gegenstand von Untersuchungen, bei denen Veränderungen des Eigenschaftsbildes wie z. B. der Leitfähigkeit, der Struktur oder der Rückstellkräfte beim Schaltprozess im Mittelpunkt standen (Li F. et al.: Polyurethane / Conducting Carbon Black Composites: Structure, Electric Conductivity, Strain Recovery Behavior, and Their Relationships. Journal of Applied Polymer Science 75 (1), 68-77 (2000)). Die Art der Zusatzstoffe, die bei den bisherigen Untersuchungen zu den Formgedächtnispolymeren hinzugegeben wurden, erstreckt sich von Kohlenstofffasern (Gall K. et al.: Carbon fiber reinforced shape memory polymer composites. Journal of Inteligent Material Systems and Structures 11 (11), 877-886 (2001); Liang C. et al.: Investigation of shape memory polymers and their hybrid composites. Journal of Inteligent Material Systems and Structures 8 (4), 380-386 (1997)) über SiC (Gall K. et al.: Shape memory polymer nanocomposites. Acta Materialia 50, 5115-5126 (2002)) bis hin zu Metallen (Gotthardt R. et al.: Smart materials based on shape memory alloys (SMAs): examples from Europe. Materials Science Forum 327-328 (Shape Memory Materials), 83-90 (2000); Monkmann G.J.: Advances in Shape Memory Polymer Actuation. Mechatronics 10, 489-498 (2000)). So soll durch Zugabe von Kohlenstofffasern zu Formgedächtnispolymeren vor allem eine erhöhte Festigkeit bzw. Steifigkeit erzielt werden. Typische Effekte bei der Zugabe von Metallen oder Metalllegierungen als Partikelzusatz zu den Formgedächtnispolymeren werden in Monkmann (s.o.) anhand von vergleichenden Untersuchungen zu partikelfreien Formgedächtnispolymeren und in Gotthardt R. et al. anhand der Dämpfung des Schwingverhaltens in Skiern diskutiert.

Den Einsatz von in Formgedächtnispolymeren eingebetteten Wärmeleitern beschreibt die Firma CTD (Lafayette, Colorado, USA), die ihr Produkt "Tembo" für den Einsatz im Weltraum konzipiert hat. Hierbei soll durch Erwärmung die Formgedächtnisfunktion als Scharnier zum Entfalten von Sonnensegeln benutzt werden, die während des Transports in den Weltraum noch zusammengefaltet vorliegen.

In eine Silikatschicht eingebettetes Eisenoxid, das sich beim Anlegen eines magnetischen Wechselfeldes aufheizt, wird seit kurzem von dem Unternehmen "Advanced Nanomaterials" angeboten.

Eine neue Möglichkeit stellt die Zumischung von ferromagnetischen oder metallischen Partikeln zu einem Formgedächtnispolymer dar (US 20050212630 A). Dabei werden die Partikel als "Antenne" für die Auslösung des Formgedächtniseffekts durch einen äußeren Stimulus genutzt. In DE 10 2007 061 342.5 sind entsprechende Mischungen von Formgedächtnispolymeren mit geeigneten Partikeln offenbart, sowie Verfahren zu deren Herstellung und thermischer Programmierung und Schaltung. Der Offenbarungsgehalt der DE 10 2007 061 342 .5 wird vollständig in die vorliegende Anmeldung aufgenommen. Hierbei ist der äußere Stimulus ein alternierendes elektromagnetisches Feld, das in den ferromagnetischen oder metallischen Partikeln Wärme erzeugt, die zu einer Temperaturerhöhung im Formgedächtnispolymer und damit zur Auslösung des Formgedächtniseffekts führt. Der Umfang des erreichbaren Temperaturanstiegs ist von vielen Parametern abhängig. Dazu gehören einerseits Parameter, die durch das EMF bestimmt sind, insbesondere Frequenz und Feldstärke des Wechselfeldes. Andererseits hängt die bei einem gegebenen Feld maximal erreichbare Materialtemperatur von der Zusammensetzung der Mischung enthaltend ein Formgedächtnispolymer und geeignete Nanopartikel selbst ab, insbesondere von der Größe, der Art, der Menge und der Wärmeleitung der Nanopartikel. Es wurde ferner festgestellt, dass auch das Oberflächen-Volumen-Verhältnis (O/V) aufgrund der von diesem abhängenden Wärmeabgabe an die Umgebung einen Einfluss auf die erreichbare Materialtemperatur hat. So lassen sich in Formgedächtniskörpern mit einem relativ geringem O/V höhere Materialtemperaturen im EMF erzielen als in solchen mit großer Oberfläche.

Dennoch ist eine Optimierung der oben genannten Parameter nicht immer ausreichend, um die erforderliche Schalttemperatur im Formgedächtniskörper zu erreichen. So können im medizinischen Bereich, wenn beispielsweise ein Formgedächtniskörper etwa als Nahtmaterial im menschlichen Körper eingesetzt wird, Frequenz oder Feldstärke des EMF nicht frei gewählt werden, um höhere Energien in den Formgedächtniskörper einzukoppeln, da sonst eine Schädigung des Körpers eintreten könnte. Auch eine Erhöhung der Konzentration der Nanopartikel ist nur in beschränktem Maße möglich, damit die mechanischen Eigenschaften des Formgedächtniskörpers weitestgehend erhalten bleiben. Schließlich unterliegt auch das Oberflächen-Volumen-Verhältnis anwendungsbedingten Grenzen.

Aufgabe der vorliegenden Erfindung ist es daher, einen im EMF schaltbaren Formgedächtniskörper zur Verfügung zu stellen, dessen gespeicherte Form auch in einer Umgebung aus Materialien mit hohen Wärmeübertragungszahlen abrufbar ist.

Die Aufgabe wird gelöst durch Bereitstellung eines Artikels mit mindestens einem Schaltabschnitt, wobei der mindestens eine Schaltabschnitt umfasst

a) einen Formgedächtniscompound, der mindestens ein thermo-programmierbares Formgedächtnispolymer und darin eingearbeitete Partikel enthält, die geeignet sind, sich in einem alternierenden elektromagnetischen Feld zu erwärmen, wobei der Formgedächtniscompound eine Wärmeübergangszahl $h_{schalt}$ aufweist;

b) einen Isolierbereich, der den Formgedächtniscompound a) umgibt und der eine Wärmeübergangszahl $h_{iso}$ aufweist;

wobei $h_{iso} < h_{schalt}$ ist.

Bevorzugt weist der Schaltabschnitt eine glatte und/oder fluidundurchlässige Oberfläche auf, die ein Eindringen einer Flüssigkeit und/oder eines Gases in den Isolierbereich verhindert.

Wesentlich für die Funktionsweise eines Schaltabschnitts ist, dass, aufgrund der Überschreitung einer Schalttemperatur $T_{sw}$ des mindestens einen thermo-programmierbaren Formgedächtnispolymers in dem Formgedächtniscompound, der Formgedächtniscompound in seine ursprüngliche, permanente Stellung zurückgeführt wird. Die dafür notwendige Temperatur lässt sich in einer Umgebung, die eine größere Wärmeübergangszahl aufweist als das Formgedächtnispolymer des Schaltabschnitts, meist nur schwer erzielen. Die in der vorliegenden Erfindung vorgestellte Lösung beinhaltet einen Isolierbereich, der den aktiven Formgedächtniscompound eines Schaltabschnitts umgibt, gleichsam wie eine Isolierschicht. Dieser Isolierbereich weist dabei eine Wärmeübergangszahl $h_{iso}$ auf, die kleiner ist als die Wärmeübergangszahl des Formgedächtniscompounds des Schaltabschnitts $h_{schalt}$. Dadurch wird die aktive Region des Schaltabschnitts in der ein Temperaturanstieg erreicht werden soll, unabhängig von der Umgebung, in der sich der Formgedächtniscompound befindet, nun von einem Bereich umgeben, der eine günstige Wärmeübergangszahl aufweist. Der aktive Bereich eines Schaltabschnitts wird somit gegen seine Umgebung isoliert. Eine Wärmeenergie, die einem Formgedächtniscompound eines Schaltabschnitts durch ein externes alternierendes EMF zugeführt wird, fließt nicht so schnell ab und es kann in dem Formgedächtniscompound insgesamt eine höhere Temperatur erreicht und somit eine kritische Schalttemperatur $T_{sw}$ des mindestens einen thermo-programmierbaren Formgedächtnispolymers leichter überschritten werden.

Der erfindungsgemäße Artikel weist mindestens einen Schaltabschnitt auf. Dabei umfasst der erfindungsgemäße Artikel jeden Gegenstand, vorausgesetzt dieser weist mindestens einen Schaltabschnitt auf.

Unter einem Schaltabschnitt wird ein Bereich des Artikels verstanden, der ein Formgedächtniscompound und einen Isolierbereich umfasst. Der Schaltabschnitt hat die Funktion, dass die Form des Schaltabschnitts und damit ggf. die Form des Artikels verändert werden kann, in dem in einem Bereich des Schaltabschnitts ein Schaltvorgang ausgelöst wird. Für die Zwecke der vorliegenden Erfindung wird unter dem Begriff "Schaltabschnitt" insbesondere ein Bereich eines Artikels verstanden, der ein Formgedächtnis aufweist, thermo-programmierbar ist und durch ein externes alternierendes elektromagnetisches Feld geschaltet werden kann, also von einer temporären in eine permanente Form überführt werden kann.

Zu diesem Zweck umfasst der Schaltabschnitt ein Formgedächtniscompound. Unter einem Formgedächtniscompound wird dabei eine Zusammensetzung verstanden, die mindestens ein thermo-programmierbares Formgedächtnispolymer und darin eingearbeitete Partikel enthält, die geeignet sind, sich in einem alternierenden elektromagnetischen Feld zu erwärmen. Daneben kann ein Formgedächtniscompound weitere Materialien aufweisen. Insbesondere kann ein Formgedächtniscompound ein oder mehrere gleiche oder verschiedene zusätzliche Formgedächtnispolymere aufweisen, die nicht alle thermo-programmierbar sein müssen. Der Formgedächtniscompound weist eine Wärmeübergangszahl $h_{schalt}$ auf.

Die Erfindung schließt auch solche Artikel ein, die insgesamt aus einem Formgedächtniscompound bestehen, der teilweise oder insgesamt umgeben von dem erfindungsgemäßen Isolierbereich ist. Dabei kann die Form des gesamten Artikels schaltbar sein.

Bei dem mindestens einen thermo-programmierbaren Formgedächtnispolymer handelt es sich um ein herkömmliches Formgedächtnispolymer oder SMP (shape memory polymer) mit thermisch stimulierbarem Formgedächtniseffekt. Das heißt es ist in der Lage, temperaturinduziert zumindest einen Formübergang von einer thermo-mechanisch programmierten temporären Form in eine permanente Form zu vollziehen. Dem Fachmann sind solche Formgedächtnispolymere bekannt.

Bei dem mindestens einen thermo-programmierbaren Formgedächtnispolymer kann es sich insbesondere um ein Polymernetzwerk mit thermisch induzierbarem Formgedächtniseffekt handeln. Die Netzwerkbildung kann dabei durch kovalente Bindungen realisiert sein oder durch physikalische Wechselwirkungen, beispielsweise elektrostatische Effekte. Neben den Vernetzungspunkten umfasst das Polymernetzwerk zumindest eine Sorte eines Schaltsegments, das eine materialabhängige Übergangstemperatur, etwa eine Kristallisationstemperatur oder Glasübergangstemperatur, aufweist. Polymernetzwerke, die einen Formgedächtniseffekt aufweisen, sind in der Literatur zahlreich beschrieben. Grundsätzlich ist die vorliegende Erfindung auf kein spezielles Material beschränkt. Beispielsweise kann das Polymernetzwerk ein Schaltsegment aufweisen, das ausgewählt ist aus der Gruppe der Polyester, insbesondere Poly($\varepsilon$-caprolacton); Polyether, Polyurethane, Polyetherurethane, Polyamide, Polyimide, Polyetherimide, Polyacrylate, Polymethacrylate, Polyvinyle, Polystyrole, Polyoxymethylene, Poly(para-dioxanon) oder andere. Denkbar ist ebenfalls, dass das Polymernetzwerk zwei oder mehr unterschiedliche Schaltsegmente aus der vorstehenden Gruppe oder andere aufweist. Dabei wird das zumindest eine Schaltsegment vorzugsweise so gewählt, dass seine Schalttemperatur in einem für die jeweilige Anwendung akzeptablen Bereich liegt.

Optional kann das Formgedächtnispolymer hydrolytisch spaltbare Gruppen aufweisen, insbesondere Ester-, Amid-, Anhydrid-, Carbonat-, Ether-, Orthoestergruppen oder Kombinationen von diesen. Auf diese Weise werden bioabbaubare Materialien erhalten, was insbesondere für Anwendungen im biomedizinischen Bereich vorteilhaft sein kann. Auch bioabbaubare Formgedächtnispolymere sind aus der Literatur hinlänglich bekannt. Die vorliegende Erfindung ist auf keine speziellen Vertreter dieser Gruppe eingeschränkt.

In das mindestens eine thermo-programmierbare Formgedächtnispolymer eines Formgedächtniscompounds sind Partikel eingearbeitet, die geeignet sind, sich in einem alternierenden elektromagnetischen Feld zu erwärmen. Dabei kann es sich um Nanopartikel oder Partikel aus dem Mikrometerbereich handeln. Es sind vorliegend Mikropartikel durch einen mittleren Partikeldurchmesser im Bereich von 1 bis 999 $\mu$m definiert und Nanopartikel durch einen mittleren Partikeldurchmesser im Bereich von 1 bis 999 nm. Diese Definition schließt somit ein, dass die Partikel als Material mit pulverförmiger Konsistenz eingebracht werden können. In stofflicher Hinsicht kommen für die Partikel alle Materialien infrage, die geeignet sind, in einem alternierenden magnetischen Feld eine Wechselwirkung zu zeigen, die zur Erwärmung der Partikel führt. Insbesondere können die Partikel Metalle umfassen, beispielsweise Ni, Fe und/oder Co. Geeignet sind darüber hinaus Legierungen, insbesondere Ni-Si, Fe-Pt, Ni-Pd und/oder Co-Pd. Weiterhin können Metalloxide als magnetisches Material in den Partikeln verwendet werden, insbesondere Ni-Zn-Fe-O, Ba-Co-Fe-O und/oder Fe-O. Daneben können Magnetite oder Eisenoxide zum Einsatz kommen, in denen die Eisenatome zumindest teilweise durch Co, Ni, Mn, Zn, Mg, Cu, Cr, Cd und/oder Ga ersetzt sind. Geeignet sind ebenfalls Ferrite, insbesondere Ni-Zn-Ferrite und/oder Sr-Ferrite. Ebenso sind Mischungen der vorgenannten Materialien möglich. Bevorzugt werden solche Materialien eingesetzt, die sich in der Polymermatrix homogen verteilen, das heißt mit dieser eine möglichst homogene Mischung ergeben. Insbesondere, wenn dieses Verhalten nicht gegeben ist, kann vorgesehen sein, dass die Partikel eine Beschichtung eines die Mischbarkeit mit dem Formgedächtnispolymer verbessernden Materials aufweisen. Als Beschichtungsmaterial kommen vor allem organische Polymere infrage. Die Natur, Größe, Anzahl und Einbettungsart richtet sich beispielsweise nach dem jeweils verwendeten Formgedächtnispolymer, der zu erreichenden Schalttemperatur und dem einzusetzenden elektromagnetischen Feld (Frequenz und Feldstärke). Bevorzugt handelt es sich um magnetische Nanopartikel mit einer durchschnittlichen Partikelgröße von < 500 nm, besonders bevorzugt von < 100 nm. Die Partikel können beispielsweise aus einem magnetischen Material bestehen oder einen schichtförmigen Aufbau aufweisen, bei dem mindestens eine Schicht die Eigenschaft aufweist, sich in einem alternierenden elektromagnetischen Feld zu erwärmen. Eine Reihe von Anbietern vertreibt geeignete Partikel und der Fachmann hat keine Schwierigkeiten diese erfindungsgemäß einzusetzen. Dabei kommt es insbesondere darauf an, dass die Nanopartikel in ein zu verwendendes Formgedächtnispolymer derart funktional eingebettet werden können, dass die Nanopartikel durch ein externes alternierendes elektromagnetisches Feld erwärmt werden können. Die Erwärmung der eingebetteten Nanopartikel führt dann zu einer Erwärmung des die Partikel umgebenden Formgedächtnispolymers.

Der mindestens eine Schaltabschnitt weist einen Aufbau auf, der so gestaltet ist, dass ein im Querschnitt des Schaltabschnitts innen liegender Formgedächtniscompound von einem relativ dazu im Querschnitt weiter außen liegenden Isolierbereich umschlossen ist. Der Isolierbereich umgibt den Formgedächtniscompound dabei vorzugsweise derart, dass der Formgedächtniscompound eines Schaltabschnitts eines Artikels keine direkte Berührung mit einem Medium hat, das den Artikel umgibt. Der Isolierbereich kann dabei direkt auf den Formgedächtniscompound aufgebracht sein. Es können aber auch weitere Schichten zwischen dem Formgedächtniscompound und dem Isolierbereich vorhanden sein. Der Isolierbereich kann aber auch direkt an den Formgedächtniscompound anschließen und mit dem Formgedächtniscompoundmaterial gefertigt sein. Insbesondere kann der Formgedächtniscompound völlig von dem Isolierbereich eingeschlossen sein. Zwischen dem Formgedächtniscompound eines Schaltabschnitts eines Artikels und weiteren an den Schaltabschnitt anschließenden Abschnitten des Artikels befindet sich bevorzugt kein Isolierbereich.

Der erfindungsgemäße Isolierbereich zeichnet sich dadurch aus, dass er eine Wärmeübergangszahl $h_{iso}$ aufweist, die kleiner ist als die Wärmeübergangszahl des Formgedächtniscompounds eines Schaltabschnitts $h_{schalt}$. Es ist $h_{iso} < h_{schalt}$.

Dabei kann $h_{iso}$ berechnet werden durch:

$$h_{iso} = 2 \, \lambda_{iso} / D_{iso+schalt} \, \ln(D_{schalt} / D_{iso+schalt})$$

wobei

$h_{iso}$ = Wärmeübergangszahl des Isolierbereichs
$\lambda_{iso}$ = spezifische Wärmeleitfähigkeit des Materials des Isolierbereichs
$D_{iso+schalt}$ = durchschnittlicher Durchmesser über den Formgedächtniscompound und den umgebenden Isolierbereich
$D_{schalt}$ = durchschnittlicher Durchmesser des Formgedächtniscompounds

Ist die Schichtdicke des Isolierbereichs klein im Verhältnis zum durchschnittlichen Durchmesser des Formgedächtniscompounds, so kann $h_{iso}$ näherungsweise berechnet werden durch:

$$h_{iso} = \lambda_{iso} / x$$

wobei

$h_{iso}$ = Wärmeübergangszahl des Isolierbereichs
$\lambda_{iso}$ = spezifische Wärmeleitfähigkeit des Materials des Isolierbereichs
x = durchschnittliche Schichtdicke des Isolierbereichs

Insbesondere ist der Isolierbereich so gestaltet, dass das Verhältnis $h_{iso} / h_{schalt} \leq 0,9$ ist, bevorzugt $\leq 0,5$, besonders bevorzugt $\leq 0,3$, ganz besonders bevorzugt $\leq 0,1$.

Der Isolierbereich kann ein oder mehrere Formgedächtnispolymere aufweisen, die auch im Formgedächtniscompound des Schaltabschnitts vorhanden sind. Der Isolierbereich kann aber auch mehrere Materialien aufweisen, die nicht im Formgedächtniscompound vorhanden sind. Insbesondere kann der Isolierbereich so gestaltet sein, dass dieser im Wesentlichen keine Partikel aufweist, die geeignet sind, sich in einem alternierenden elektromagnetischen Feld zu erwärmen. Der Isolierbereich kann ein Material aufweisen oder aus diesem bestehen, dessen spezifische Wärmeleitfähigkeit $\lambda_{iso}$ geringer ist als die spezifische Wärmeleitfähigkeit $\lambda_{schaft}$ eines Materials des Formgedächtniscompounds, bevorzugt des mindestens einen thermo-programmierbaren Formgedächtnispolymers des Formgedächtniscompounds.

Bei dem Material mit der geringeren spezifischen Wärmeleitfähigkeit $\lambda_{iso}$ des Isolierbereichs kann es sich insbesondere um ein Gas handeln. Bevorzugt wird ein Gas verwendet, welches sich inert verhält gegenüber anderen Materialien des Isolierbereichs und/oder des Formgedächtniscompounds. Besonders bevorzugt handelt es sich bei dem Gas um $CO_2$ oder Luft oder Gemische enthaltend eines dieser Gase in einer Konzentration von $\geq 10$ Masse-%. Insbesondere wenn der Isolierbereich eine Schaumstruktur aufweist, können Teile der oder alle Zellen einer Schaumstruktur ein solches Gas mit entsprechender spezifischer Wärmeleitfähigkeit enthalten.

Um eine besonders effektive Isolierung des Formgedächtniscompounds des Schaltabschnitts zu erreichen, kann der Isolierbereich eine Strukturierung aufweisen, die sich von der Strukturierung des Formgedächtniscompounds unterscheidet. Dabei kann der Isolierbereich beispielsweise aus mehreren gleichen oder verschiedenen Schichten aufgebaut sein oder eine poröse Struktur aufweisen. Dabei kann die poröse Struktur im Wesentlichen offenzellig oder geschlossenzellig aufgebaut sein. Weist der Isolierbereich eine poröse Struktur auf, so können Teil oder alle Hohlräume der porösen Struktur mit einem Material gefüllt sein, das eine spezifische Wärmeleitzahl aufweist, die geringer ist als die spezifische Wärmeleitzahl eines thermo-programmierbaren Formgedächtnispolymers des Formgedächtniscompounds des Schaltabschnitts. Insbesondere kann der Isolierbereich in Teilen oder im Ganzen eine Schaumstruktur aufweisen, insbesondere eine vorwiegend geschlossenzellige Schaumstruktur. Der Isolierbereich kann Poren aufweisen, die entlang eines Porengrößengradienten über den Querschnitt des Isolierbereichs verteilt vorliegen, wobei die Poren in einem äußeren Bereich des Isolierbereichs im Durchschnitt kleiner sind als die Poren in einem inneren Bereich des Isolierbereichs. In einer bevorzugten Ausführungsform weist der Isolierbereich Poren und/oder Schaumzellen mit einer durchschnittlichen Größe von 5 bis 50 $\mu$m auf.

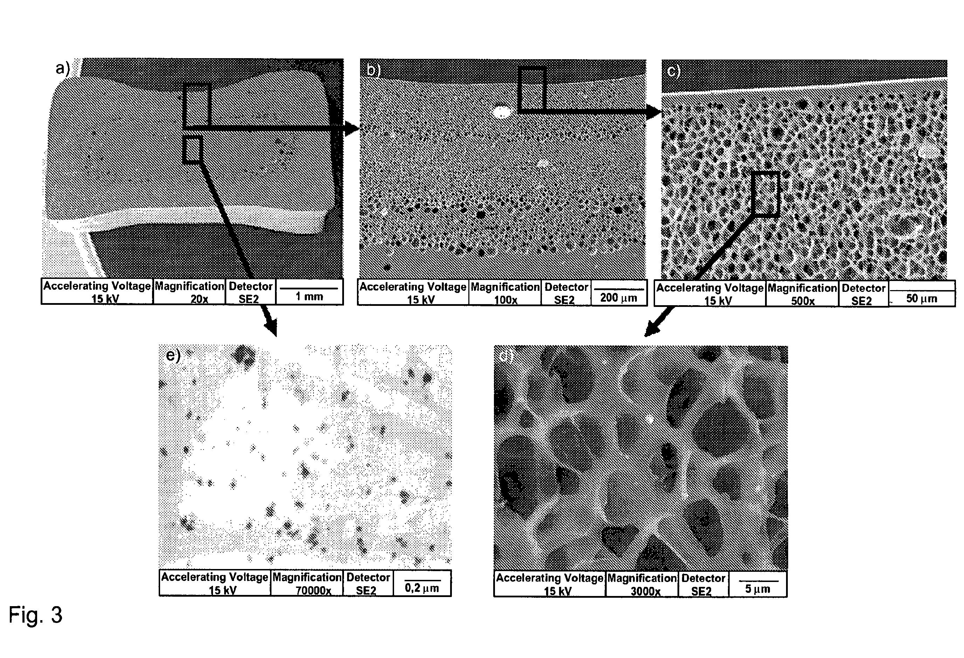

Insbesondere kann der Schaltabschnitt, bevorzugt der Isolierbereich, eine glatte äußerste Oberfläche aufweisen (siehe auch FIG. 3 a) bis c)). Die glatte Oberfläche des Schaltabschnitts und/oder des Isolierbereichs hat die Funktion eine mechanische Stabilität des Formgedächtniscompounds zu gewährleisten und zu verhindern, dass andere Materialien wie beispielsweise Flüssigkeiten oder Gase in das innere des Formgedächtniscompounds eindringen können.

Insbesondere kann der Isolierbereich eine durchschnittliche Schichtdicke aufweisen, die dazu führt, dass der Formgedächtniscompound effektiv isoliert wird von einer Umgebung, die den Schaltabschnitt umgibt. Ist dem Fachmann bekannt, in welcher Umgebung der Schaltabschnitt durch ein externes elektromagnetisches Feld geschaltet werden soll, und ist dem Fachmann bekannt, welches thermo-programmierbare Formgedächtnispolymer im Formgedächtnispolymer des Schaltabschnitts mit Partikeln versetzt ist und geschaltet werden soll, so kann der Fachmann die notwendige durchschnittliche Schichtdicke des Isolierbereichs für ein gegebenes EMF leicht bestimmen. Bestehen also Beschränkungen bzgl. des EMF, so kann der Einsatz eines schwächeren Feldes ggf. dadurch ausgeglichen werden, dass der Isolierbereich eine bestimmte Dicke aufweist und/oder bestimmte Materialien aufweist, die eine besonders günstige spezifische Wärmeleitfähigkeit besitzen.

Insbesondere kann das Verhältnis des gemittelten Durchmessers des Isolierbereichs und des Formgedächtniscompounds ($D_{iso+schalt}$) zu dem gemittelten Durchmesser des Formgedächtniscompounds ($D_{schalt}$) im Querschnitt des mindestens einen Schaltabschnitts $D_{iso+schalt}$ / $D_{schalt}$ so gewählt sein, dass im Formgedächtniscompound durch Anregung mit einem alternierenden elektromagnetischen Feld eine Schalttemperatur des mindestens einen thermo-programmierbaren Formgedächtnispolymers des Formgedächtniscompounds erreichbar ist, wenn sich der mindestens eine Schaltabschnitt in einer Umgebung mit einer Wärmeübergangszahl befindet, die größer ist, als die Wärmeübergangszahl des Formgedächtniscompounds des Schaltabschnitts. Dabei kann das Durchmesserverhältnis $D_{iso+schalt}$ / $D_{schalt}$ so gewählt sein, dass der mindestens eine Schaltabschnitt in einer wässrigen Umgebung oder einer Umgebung aus Wasser und/oder in einem alternierenden elektromagnetischen Feld mit einer Frequenz von 245 bis 265 kHz und einer Feldstärke von 10 bis 16 kA/m die Schalttemperatur erreicht.

Das Verhältnis des gemittelten Durchmessers des Isolierbereichs und des Formgedächtniscompounds zu dem gemittelten Durchmesser des Formgedächtniscompounds im Querschnitt des mindestens einen Schaltabschnitts kann insbesondere $D_{iso+schalt}$ / $D_{schalt} \geq 1,01$ sein, bevorzugt $\geq 1,05$, besonders bevorzugt $\geq 1,1$.

Die Funktion des Isolierbereichs ist es, eine Wärmeübertragung von dem Artikel in eine Umgebung im Bereich des Schaltabschnitts zu vermindern, während in einem Formgedächtniscompound des Schaltabschnitts über die eingebetteten Nanopartikel eine Erwärmung des Körpers in einem elektromagnetischen Feld bewirkt werden kann, ohne dass es zu einem signifikanten Wärmeabfluss in die Umwelt des Artikels kommt.

Der Schaltabschnitt kann mit Vorteil hierarchisch strukturiert werden, d.h. neben der Struktur des Formgedächtniscompounds auf der nano/mikro-Ebene (Hart- und Schaltdomäne/Nanopartikel) wird eine weitere Organisation im $\mu$m/mm-Bereich eingeführt, die Strukturierung in Isolierbereich und Formgedächtniscompound eines Schaltabschnitts. Diese Makrostrukturierung wird so gestaltet, dass eine Isolation zwischen Umgebungsmedium und Formgedächtniscompound eines Schaltabschnitts des Artikels erreicht und somit eine Verminderung der Wärmübergangszahl realisiert wird.

Die Realisierung eines Isolierbereichs oder "Mantels" der nur kleine Wärmeübergangzahlen zulässt, führt dazu, dass die Größe des Temperatureffektes im Formgedächtniscompound, der durch ein extern wirkendes alternierendes EMF hervorgerufen wird, nun auch in fluiden Anwendungsbereichen oder Gebieten mit großer Wärmübergangszahl ausreichend ist, um einen Formgedächtniseffekt zu erzielen.

Das speziell strukturierte Material kann sowohl während der Herstellung des Formgedächtniscompounds, als auch in einem nachgelagerten Herstellungsprozess realisiert werden.

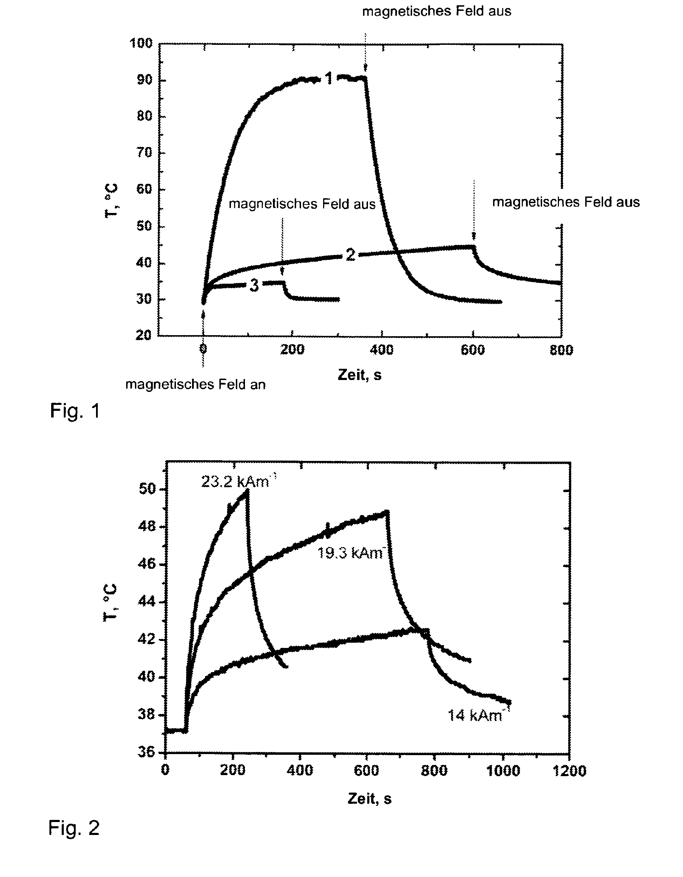

Die Erfindung macht beispielsweise von der Erkenntnis Gebrauch, dass der maximal erreichbare Temperaturanstieg des Materials neben den oben genannten Parametern auch stark von der Art des Einsatzgebietes abhängt. Denn die Wärmeübergänge zum benachbarten Materialien beeinflussen ebenfalls die maximal erreichbaren Temperaturdifferenzen. Eine besondere Rolle spielt die Wärmeabführung (ausgedrückt als Wärmeübergangszahl) an die Umgebung des Formgedächtniscompounds. Dabei wird irgendwann ein Gleichgewicht zwischen der über das EMF zugeführte und durch die Nanopartikel an die Umgebung abgeführte Wärmeenergie erreicht, welches in einer maximal erreichbaren Temperatur resultiert. Bei Kontakt des Formgedächtniscompounds mit Materialien mit hohen Wärmeübergangszahlen ist die erzielbare Temperaturdifferenz deutlich geringer als bei Kontakt mit Materialien mit kleinen Übergangszahlen bei ansonsten vergleichbaren Bedingungen. Daher reicht in erstem Fall oftmals die zugeführte Energie nicht aus, um Temperaturen zu erreichen, die einen thermischen Formgedächtniseffekt auslösen. Dies wird durch FIG. 1 und FIG. 2 deutlich unterstrichen.

Eine erfindungsgemäße Nutzung der indirekten Anregung von nanoskaligen Partikeln in Formgedächtniscompounds durch ein extern wirkendes EMF und die damit verbundene Temperaturerhöhung bzw. Auslösung des Formgedächtniseffektes im medizinischen Bereich lässt die Vorteile besonders deutlich hervortreten. Wenn der Formgedächtniscompound z.B. als Nahtmaterial im menschlichen Körper eingesetzt wird, ergeben sich zwangsläufig hohe Wärmeübergangszahlen zum umgebenden Gewebe. Um den beschriebenen Formgedächtniseffekt z.B. auch unter solchen Bedingungen realisieren zu können, wird die erfindungsgemäße Lösung vorgeschlagen.

Die Erfindung bezieht sich auch auf erfindungsgemäße Artikel für Anwendungen, bei denen der Artikel in Kontakt kommt mit Materialien mit einer Wärmeübergangszahl, die größer ist als $h_{schalt}$, insbesondere mit flüssigen Materialien oder Geweben des menschlichen oder tierischen Körpers. Beispiele für solche Artikel, die mit Materialien mit einer Wärmeübergangszahl, die größer als $h_{schalt}$ ist, in Kontakt kommen, sind Katheter, wie z.B. steuerbare oder expandierbare Katheter, Gefäßstützen, wie z.B. selbstexpandierbare und/oder entfernbare Gefäßstützen und/oder Stents, insbesondere für den Koronarbereich, die Blase oder die Nieren oder Venenfilter, wie Vena-Cava-Filter.

Die Erfindung bezieht sich insbesondere auch auf erfindungsgemäße Artikel zur Anwendung in einem Verfahren zur chirurgischen oder therapeutischen Behandlung des menschlichen oder tierischen Körpers oder in einem Diagnostizierverfahren, wobei der Artikel in Kontakt gebracht wird mit Bestandteilen eines menschlichen oder tierischen Körpers. Beispiele für Artikel zur Anwendung in einem Verfahren zur chirurgischen oder therapeutischen Behandlung sind beispielsweise Drug-delivery-Systeme, insbesondere implantierbare und extern steuerbare Drug-delivery-Systeme, medizinische Geräte, wie z.B. für die minimal invasive Chirurgie optimierte medizinische Geräte, wie z.B. guide wires, insbesondere steuerbare guide wires, oder Nahtmaterialien.

Die Erfindung bezieht sich ferner auch auf die Verwendung der erfindungsgemäßen Artikel im Bereich der Medizin, insbesondere die Anwendung in einem Verfahren zur chirurgischen oder therapeutischen Behandlung des menschlichen oder tierischen Körpers oder in einem Diagnostizierverfahren, wobei der Artikel mit Bestandteilen eines menschlichen oder tierischen Körpers in Kontakt gebracht wird. Geeignete Verwendungen für den erfindungsgemäßen Artikel sind beispielsweise die Erzeugung von stimulierbaren Knoten, Fixierungen oder Implantaten oder die operationsfreie bzw. postoperative Veränderung von Implantaten, wie beispielsweise die Anpassung bezüglich der Lage, Größe, Funktion, des Durchflusses oder des Strömungswiderstandes. Nachträgliche, d.h. postoperative Veränderungen der Lage kommen beispielsweise bei Elektroden von Herzschrittmachern zum Einsatz. Unter Änderungen der Größe wird erfindungsgemäß eine Änderung eines beliebigen Parameters der Geometrie, wie eine Änderung der Länge, Breite, Höhe, des Durchmessers oder der allgemeinen Form verstanden. Diese Anwendung der erfindungsgemäßen Artikel ist insbesondere bei Implantaten oder Prothesen in Wachstumszonen des Körpers interessant, da mittels der erfindungsgemäßen Artikel beispielsweise Gelenkprothesen und/oder Knochenersatzimplantate auf das Wachstum von Mensch und/oder Tier angepasst werden können. Unter Funktionsänderungen wird beispielsweise das Aufspannen eines Venenfilters, wie für die Vena Cava oder die Expansion von Gefäßstützen und/oder Stents verstanden. Dabei können mit Hilfe der erfindungsgemäßen Artikel sogar bestimmte Organfunktionen, beispielsweise durch eine stimulierbare Anpassung der Blutversorgung gesteuert werden. Des Weiteren sind die erfindungsgemäßen Artikel auch geeignet, um ein oder mehrere Diagnosefunktionen eines implantierbaren Überwachungssystem auf- und abzurufen. Die genannten Anwendungen und Einsatzmöglichkeiten stellen nur ausgewählte Beispiele aus dem medizinischen Anwendungsbereich, insbesondere dem Bereich der regenerativen Medizin, dar und sollen daher nicht limitierend wirken. Weitere geeignete Einsatzgebiete und Möglichkeiten sind dem Fachmann bekannt.

Die Erfindung bezieht sich auch auf ein Verfahren zur Herstellung eines erfindungsgemäßen Artikels mit mindestens einem Schaltabschnitt, umfassend die Schritte:

a) Darstellung eines Formgedächtniscompounds (des mindestens einen Schaltabschnitts), der mindestens ein thermo-programmierbares Formgedächtnispolymer und darin eingearbeitete Partikel enthält, die geeignet sind, sich in einem alternierenden elektromagnetischen Feld zu erwärmen, wobei der Formgedächtniscompound eine Wärmeübergangszahl $h_{schalt}$ aufweist; und
b) Aufbringen eines Isolierbereichs, der eine Wärmeübergangszahl $h_{iso} < h_{schalt}$ aufweist, auf den Formgedächtniscompound des mindestens einen Schaltabschnitts aus Schritt a), sodass der Isolierbereich den Formgedächtniscompound umgibt;
wobei Schritt b) gleichzeitig mit Schritt a) oder nachgelagert zu Schritt a) ausgeführt wird.

Grundsätzlich kann der Formgedächtniscompound durch dem Fachmann bekannte Verfahren und Technologien hergestellt werden, wie z.B. Verformung aus der Polymerschmelze, kontinuierliches und/oder diskontinuierliches Ein- oder Mehrschichtextrusionsverfahren, kontinuierliches und/oder diskontinuierliches Ein- oder Mehrschichtspritzgussverfahren oder durch einen Fügeprozess, bei dem mehrere vorgefertigte Teile miteinander verbunden werden. Insbesondere in DE 10 2007 061 342.5 werden geeignete Formgedächtnispolymere detailliert beschrieben, in die Partikel eingebettet sind, die geeignet sind, sich in einem alternierenden elektromagnetischen Feld zu erwärmen, sowie Verfahren zur Herstellung solcher Polymere und Verfahren zur Herstellung von Formgedächtniscompounds enthaltend solche Polymere. Bevorzugt wird der Formgedächtniscompound des mindestens einen Schaltabschnitts durch Verformung aus einer Polymerschmelze, kontinuierliches und/oder diskontinuierliches, ein- und/oder mehrschichtiges Extrusions- oder Spritzgussverfahren und/oder durch einen Fügeprozess mehrerer vorgefertigter Elemente hergestellt wird.

Der Isolierbereich kann durch mehrere verschiedene dem Fachmann aus anderen Zusammenhängen bekannte Fertigungsverfahren auf den Formgedächtniscompound eines Schaltabschnitts aufgebracht werden. Dazu eignen sich insbesondere Verschäumungsprozesse, Schichtungsverfahren (beispielsweise Air brusch-Prozesse) oder Phaseninversionsprozesse. Bei dem Verschäumungsprozess wird Gas (vorzugsweise Kohlendioxid) im überkritischen Zustand bei hohem Druck und hoher Temperatur in die Polymerschmelze eingebracht und unter diesen Bedingungen im Gleichgewicht gehalten. Nach Absenkung der Temperatur und/oder des Druckes kommt es zur spontanen Entspannung und zum Verschäumungsprozess, dessen Resultat poröse Morphologien im Formkörper erzeugen. Dieses "Verschäumen" kann kontinuierlich bei der Extrusion oder nachgelagert in einem diskontinuierlich ablaufenden Prozess (Pressure Quench) realisiert werden. Eine vollständige Verschäumung des Formgedächtniscompounds wird erreicht, sobald sich der Gleichgewichtszustand über den gesamten zu verschäumenden Körper eingestellt hat. Durch Entspannung des Gases kann der Prozess vorzeitig abgebrochen und somit eine partielle Verschäumung erreicht werden. Mit zunehmender Dauer des Verschäumungsvorgangs erfolgt die Isolierung eines erfindungsgemäßen Artikels von außen nach innen bis zur vollständigen Umhüllung.

Die Erzeugung poröser Strukturen ist aber auch durch Phaseninversionsprozesse möglich. Hierbei wird die flüssige Phase eines Polymers (Polymerlösung bzw. Polymerschmelze) einer Phaseninversion unterzogen, die durch Abkühlung der Polymerflüssigkeit bzw. durch Einwirkung eines Nichtlösemittels hervorgerufen wird.

Es können aber auch einfach Schichten des Isolierbereichs beispielsweise durch Sprühen (ggf. im Airbrush-Verfahren) auf die entsprechenden Stellen des Formgedächtniscompounds aufgebracht werden.

Bevorzugt wird der Isolierbereich durch Verschäumen, durch Sprühen und/oder durch Phaseninversion aufgebracht, insbesondere kann der Isolierbereich durch Verschäumen und/oder Phaseninversion des mindestens einen thermo-programmierbares Formgedächtnispolymers des Formgedächtniscompounds erzeugt werden. Besonders bevorzugt wird der Isolierbereich durch Verschäumen und/oder Phaseninversion eines oder mehrerer Materialien erzeugt, die sich vom mindestens einen thermo-programmierbaren Formgedächtnispolymer des Formgedächtniscompounds unterscheiden.

Beispielsweise sind folgende Verfahrensweisen von der erfindungsgemäßen Lösung umfasst:

i) Die erfindungsgemäße Lösung kann dadurch erreicht werden, dass der Formgedächtniscompound nur aus einem einzigen Material besteht.

ii) Die erfindungsgemäße Lösung kann dadurch erreicht werden, dass der Formgedächtniscompound aus mehreren verschiedenen Materialien, mindestens aber aus zwei verschiedenen Materialien besteht, wovon mindestens eines ein thermo-programmierbares Formgedächtnispolymer darstellt.

iii) Die erfindungsgemäße Lösung kann dadurch erreicht werden, dass der Formgedächtniscompound durch kontinuierliche Extrusion erzeugt und durch gleichzeitig partielles Verschäumen der Materialien eine integral nichteinheitliche Morphologie über den Querschnitt erzielt wird.

iv) Die erfindungsgemäße Lösung kann dadurch erreicht werden, dass der Formgedächtniscompound durch Extrusion erzeugt und in einem nachgelagerten "Pressure Quench" Prozess durch partielles Verschäumen der Materialien eine integral nichteinheitliche Morphologie über den Querschnitt erzielt wird.

v) Die erfindungsgemäße Lösung kann dadurch erreicht werden, dass der Formgedächtniscompound durch Extrusion erzeugt und in einem nachgelagerten partiellen Phaseninversionsprozess der Materialien eine integral nichteinheitliche Morphologie über den Querschnitt erzielt wird.

vi) Die erfindungsgemäße Lösung kann dadurch erreicht werden, dass der Formgedächtniscompound durch kontinuierliche Mehrschichtextrusion erzeugt und gleichzeitig durch partielles Verschäumen der Materialien eine integral nichteinheitliche Morphologie über den Querschnitt erzielt wird.

vii) Die erfindungsgemäße Lösung kann dadurch erreicht werden, dass der Formgedächtniscompound durch Mehrschichtextrusion erzeugt und in einem nachgelagerten "Pressure Quench" Prozess durch partielles Verschäumen der Materialien eine integral nichteinheitliche Morphologie über den Querschnitt erzielt wird.

viii) Die erfindungsgemäße Lösung kann dadurch erreicht werden, dass der Formgedächtniscompound durch Mehrschichtextrusion erzeugt und in einem nachgelagerten partiellen Phaseninversionsprozess der Materialien eine integral nichteinheitliche Morphologie über den Querschnitt erzielt wird.

ix) Die erfindungsgemäße Lösung kann dadurch erreicht werden, dass der Formgedächtniscompound durch einen Fügeprozess hergestellt wird und in einem nachgelagerten "Pressure Quench" Prozess durch partielles Verschäumen eine integral nichteinheitliche Morphologie über den Querschnitt erzielt wird.

x) Die erfindungsgemäße Lösung kann dadurch erreicht werden, dass der Formgedächtniscompound durch einen Fügeprozess hergestellt wird und in einem nachgelagerten partiellen Phaseninversionsprozess eine integral nichteinheitliche Morphologie über den Querschnitt erzielt wird.

Im erfindungsgemäßen Verfahren kann der Isolierbereich durch Verschäumen und/oder Phaseninversion eines Formgedächtnispolymers des Formgedächtniscompounds erzeugt werden.

Im erfindungsgemäßen Verfahren kann der Isolierbereich beispielsweise auch durch Verschäumen und/oder Phaseninversion eines oder mehrerer Materialien erzeugt werden, die sich vom Formgedächtnispolymer des Formgedächtniscompounds unterscheiden.

Zur besseren Verdeutlichung dieses speziell strukturierten Materials ist in FIG. 3 ein Beispiel für einen beispielhaften morphologischen Aufbau dargestellt.

Des weiteren bezieht sich die Erfindung auf einen Artikel aus einem Formgedächtniscompound, der ein Formgedächtnispolymer mit einer Hartdomäne und mindestens einer Schaltdomäne umfasst sowie in dem Formgedächtnispolymer eingearbeitete Partikel, die geeignet sind, sich in einem alternierenden elektromagnetischen Feld zu erwärmen, dadurch gekennzeichnet, dass der Artikel einen Aufbau aufweist, der so gestaltet ist, dass ein im Querschnitt des Körpers relativ äußerer Bereich geringere Wärmeübergangszahlen aufweist als ein im Querschnitt des Körpers relativ innerer Bereich. Insbesondere kann der relativ äußere Bereich eine Strukturierung aufweisen, welche die geringeren Wärmeübergangszahlen bedingt, insbesondere eine Schaumstruktur. Bevorzugt kann der relativ äußere Bereich ein Material aufweisen, dessen spezifische (massebezogene) Wärmeübergangszahl kleiner ist als die spezifische Wärmeübergangszahl des Material des relativ inneren Bereichs.

Die vorliegende Erfindung bezieht sich auch auf ein Verfahren zur Herstellung eines erfindungsgemäßen Formgedächtniscompounds, wobei eine Formmasse, die ein Formgedächtnispolymer mit einer Hartdomäne und mindestens einer Schaltdomäne umfasst sowie in dem Formgedächtnispolymer eingearbeitete Partikel, die geeignet sind, sich in einem alternierenden elektromagnetischen Feld zu erwärmen, mit einem Treibmittel vermischt und in einem Spritzgussverfahren partiell aufgeschäumt wird.

Die Erfindung wird nachfolgend in Ausführungsbeispielen anhand der zugehörigen Zeichnungen erläutert. Es zeigen:

FIG. 1 Erwärmungskurven für Formgedächtniscompounds (18,1 % Nanopartikel in Formgedächtnispolymer) in unterschiedlicher Umgebung, 1 - Luft, 2 - 10 % Gelatine in 0,5 %iger NaCl Lösung, 3 - destilliertes Wasser;

FIG. 2 Erwärmungskurven für ein Formgedächtniscompound (14,4 % Nanopartikel in Formgedächtnispolymer mit einem Oberflächen/Volumen Verhältnis von 8,3) in Schweinefleisch bei verschiedenen Magnetfeldstärken;

FIG. 3 a)-e) Morphologischer Aufbau einer Ausführungsform eines erfindungsgemäßen Formgedächtniscompounds mit einer speziell strukturierten Schichtung;

FIG. 4 Zylinderförmige Ausführungsform eines erfindungsgemäßen Artikels (a) mit einer Ausschnittvergrößerung aus dem Kernbereich (b);

FIG. 5 Schematischer Versuchsaufbau zur Ermittlung des Temperaturverhaltens unterschiedlicher Proben im magnetischen Wechselfeld in einem Medium mit hoher Wärmeübergangszahl;

FIG. 6 Temperaturverlauf von a) einem Polymerzylinder in Wasser mit einem verschäumten Isolierbereich bei EFM unterschiedlicher Feldstärke und von b) einem Vergleich zwischen einem Polymerzylinder mit oder ohne einem verschäumten Isolierbereich;

FIG.7 Temperaturverlauf von unterschiedlich strukturierten Formgedächtniscompounds im EMF in unterschiedlichen Medien (Wasser, Luft);

FIG. 8 Diagramm zur Darstellung des Verschäumungsprozesses;

FIG. 9 Schematische Darstellung einer Expansion eines erfindungsgemäßen künstlichen Blutgefäßes aus einem Formgedächtniscompound (a) und elektronenmikroskopische Aufnahme eines solchen in Längsrichtung (b); und

FIG. 10      Schematische Darstellung der Verschäumung eines erfindungsgemäßen Artikels bei röhrenförmiger (a) und zylindrischer (b) Geometrie.

Ausführungsbeispiele:

Beispiel 1:

Ein Formgedächtnispolymer enthaltend Nanopartikel (MagSilica 50-85) wird durch ein Spritzgussverfahren zu einem Zylinder mit einem Durchmesser von 5 mm und einer Höhe von 5 mm verformt. Dieser Zylinder wird in einem nachgelagerten Prozessschritt in einem Autoklaven teilweise verschäumt. Hierbei erfolgt eine Erhöhung der Porosität in der äußeren Zylinderschicht durch den Kontakt des Polymermaterials mit Kohlendioxid (überkritisch) bei erhöhten Druck (100 bar) und hoher Temperatur (100°C). Nach 5 min (Sättigungsphase) erfolgt eine spontane Druckabsenkung mit einer Geschwindigkeit von 900 bar/min und Abkühlung (Raumtemperatur) im Autoklaven. Der dabei entstehende aufgeschäumte Isolierbereich weist eine durchschnittliche Schichtdicke von ca. 0,4 mm auf.

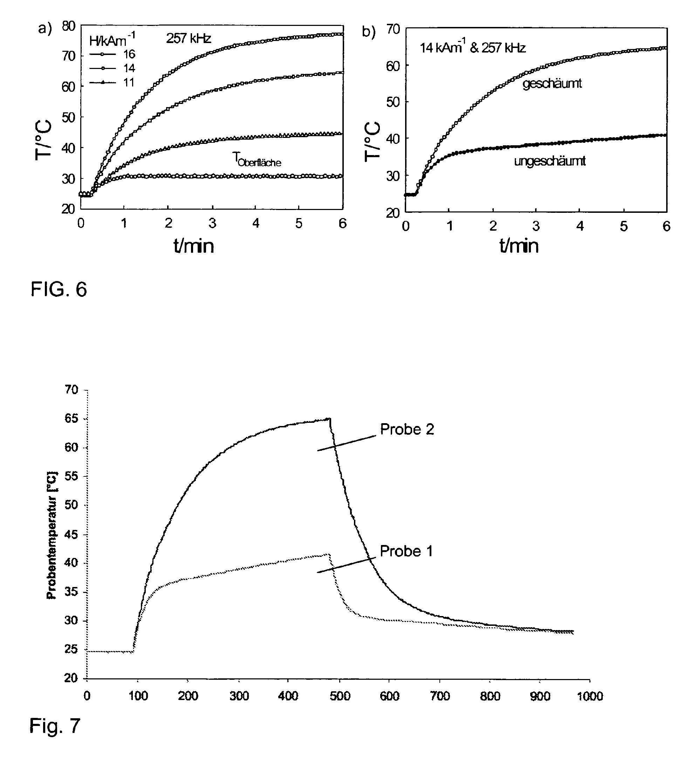

Ein diesem zweiten Prozessschritt nicht ausgesetzter Polymerzylinder (Probe 1) und ein diesem zweiten Prozessschritt ausgesetzten Polymerzylinder mit einem porösen äußeren Isolierbereich (Probe 2) sind Ausgangspunkt des Ausführungsbeispiels. Bei beiden Proben besteht die Möglichkeit, die Temperatur im Inneren des Probenkörpers durch ein Thermoelement zu messen. Die Ausgangsprobe (Probe 1) und die dem erfindungsgemäßen Verfahren entsprechende Probe 2 werden einem alternierenden EMF bei einer Frequenz von 253 kHz und einer Feldstärke von 12,6 kA/m ausgesetzt. Die Messung der Temperaturerhöhung erfolgt nach 380 Sekunden jeweils in den Probekörpern, die in Wasser (große Wärmeübergangszahl) positioniert wurden. Probe 1 wies eine Innentemperatur von 42°C auf und die erfindungsgemäße Probe 2 eine Temperatur von 65°C. FIG. 7 verdeutlicht den erfindungsgemäßen Effekt.

Unter analogen Voraussetzungen kann durch erfindungsgemäße Lösungen - besonders bei einem umgebenden Medium mit hohen Wärmeübergangszahlen - eine wesentlich höhere Temperatur erzeugt werden.

Beispiel 2:

Ein Polymerkörper aus dem Formgedächtnispolymer Poly(etherurethan) mit 5 Masse-% eingearbeiteten Nanopartikeln (Eisenoxid) wurde hergestellt. Der Körper wurde einem Verschäumungsprozess mit überkritischem $CO_2$ zugeführt, bei dem ein Isolierbereich im Querschnitt des Körpers entstanden ist, der eine Schaumstruktur aufweist (siehe FIG. 3 a) bis d)). Der Isolierbereich mit der Schaumstruktur weist im Querschnitt des Körpers eine Dicke von ca. 0,4 mm auf und befindet sich an der äußeren Oberfläche des Zylinders. Der Durchmesser der Schaumzellen liegt im Bereich von 5 bis 50 $\mu$m und ist abhängig von dem Abstand der Zelle zur Oberfläche des Körpers. Je näher die Schaumzelle zur Oberfläche des Körpers liegt, desto kleiner ist ihr Durchmesser und umgekehrt. Über eine Steuerung des Druckabsenkungsschritts lassen sich Porengrößen der Schaumstruktur im Isolierbereich des Körpers steuern. FIG. 3e) zeigt, dass die Nanopartikel, hier Eisenoxidpartikel, gut verteilt in der Polymermatrix vorliegen. Insbesondere ist es erwähnenswert, dass der Isolierbereich eine glatte äußerste Oberfläche aufweist (siehe FIG. 3 a) bis c)). Die glatte fluidundurchlässige Oberfläche des Isolierbereichs hat die Funktion, eine mechanische Stabilität des Körpers zu gewährleisten und zu verhindern, dass andere Materialien wie beispielsweise Flüssigkeiten oder Gase in das Innere des Körpers eindringen können. Die Funktion des Isolierbereichs des Körpers ist es, die Wärmeübertragung des Körpers im Randbereich zu vermindern, während in einem Formgedächtniscompound des Körpers über die eingebetteten Nanopartikel eine Erwärmung des Körpers in einem elektromagnetischen Feld bewirkt werden kann, ohne dass es zu einem signifikanten Wärmeabfluss in die Umwelt des Körpers kommt.

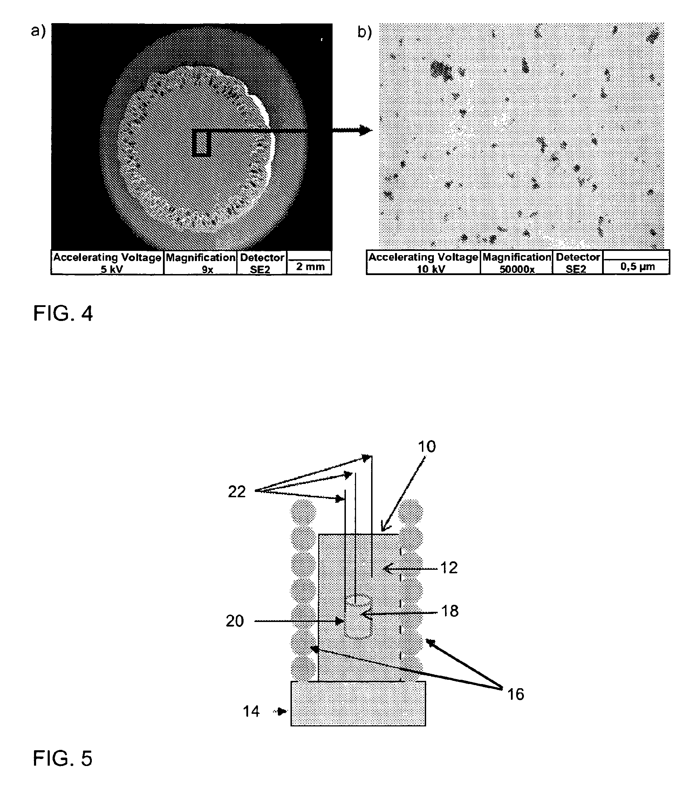

Um die Effizienz eines solchen verschäumten Isolierbereichs als Isolator zu demonstrieren, wurde ein Polymerzylinder hergestellt (Poly(etherurethan) mit 5 Masse-% Nanopartikel), der einen Durchmesser von $D_{iso+schalt}$ von 7 mm aufwies sowie einen Isolierbereich, der einen Formgedächtniscompound mit einem Durchmesser von $D_{schalt}$ von 6mm aufwies und eine Länge von 12 mm (FIG. 4 a)). FIG. 4 b) zeigt eine Ausschnittsvergrößerung aus dem Kernbereich der Poly(etherurethan)-Matrix, wobei die schwarzen Bereiche die nanopartikulären Eisenoxidteilchen darstellen.

Diese zylindrische Probe wurde gemäß Figur 5 in ein Glasgefäß 10 (Durchmesser 25 mm, Höhe 55 mm) verbracht, welches mit destilliertem Wasser 12 gefüllt war. Das Glasgefäß 10 war auf einer isolierenden Kunststoffunterlage 14 positioniert und von Spulen 16 eines Induktors umgeben. Der Induktor selbst ist nicht gezeigt. Die Temperatur in der Probe 18, der Schaumhülle 20 und in dem umgebenden Wasser 12 wurde über unterschiedliche Temperatursensoren 22 bestimmt. FIG. 5 zeigt die schematische Darstellung des Versuchsaufbaus. Die Anordnung wurde einem alternierenden elektromagnetischen Feld mit einer Frequenz von 257 kHz und unterschiedlicher Feldstärke ausgesetzt. Wie in FIG. 6a) dargestellt konnte der Zylinder 18 mit zunehmender Feldstärke schneller und insgesamt auf eine höhere Maximaltemperatur erwärmt werden. Die Temperatur an der Oberfläche des verschäumten Zylinders blieb im Wesentlichen stabil bei 30°C. Daneben konnte im Wesentlichen keine Temperaturveränderung in dem den Zylinder umgebenden Wasser nachgewiesen werden.

Anschließend wurde die Temperaturveränderung in einem Zylinder mit verschäumtem Isolierbereich bei einem konstanten Feld mit einer Frequenz von 257 kHz und einer Feldstärke von 14 kA/m gemessen und mit der Temperaturkurve eines baugleichen Zylinders verglichen, der keinem Verschäumungsprozess unterzogen wurde und somit keinen entsprechenden Isolierbereich im Sinne der Erfindung aufwies. Wie FIG. 6b) zu entnehmen ist, nimmt die Temperatur in dem Zylinder mit Isolierbereich sehr viel schneller zu und erreicht auch einen höheren Maximalwert als bei dem Vergleichszylinder.

Beispiel 3:

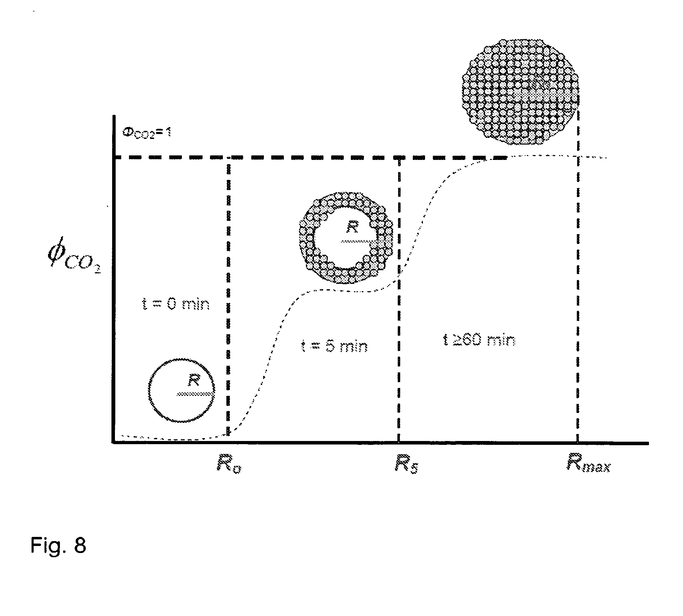

Der Verschäumungsprozess eines kugelförmigen Körpers mit dem Radius R ist in FIG. 8 schematisch dargestellt. Aufgetragen ist der Anteil an $CO_2$ im Gleichgewicht über den Radius R. Bei t = 0 min ist der gesamte Körper noch nicht verschäumt. Bei $(CO_2)$ = 1 hat sich der Gleichgewichtszustand über den gesamten Radius R eingestellt, wobei die Einstellung des Gleichgewichtes von außen nach innen erfolgt. Nach dem Entspannen weist der Körper dann eine vollständige Schaumstruktur auf. Der vollständige Gleichgewichtszustand $(CO_2)$ = 1 ist nach 60 min erreicht. Bevor sich der Gleichgewichtszustand über dem gesamten Körper eingestellt hat, kann der Verschäumungsprozess jeder Zeit, beispielsweise nach 5 min, durch Entspannung abgebrochen werden. Nach 5 min hat sich über einen bestimmten Teil des Radius, hier $R_5$, bereits das Gleichgewicht eingestellt. Nach dem Entspannen weist dieser Bereich von $R_5$ die isolierende Verschäumung auf. Mit einer geeigneten Prozessführung und dem rechtzeitigen Abbruch des Verschäumungsprozesses kann somit eine partielle Verschäumung erreicht werden.

Beispiel 4:

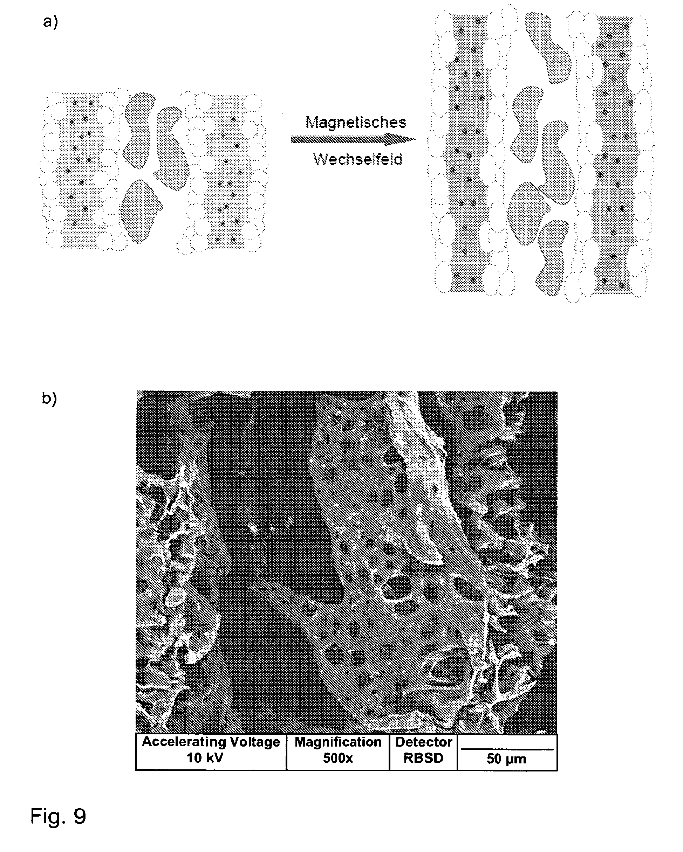

Ein erfindungsgemäßer Formgedächtniscompound wird als Gefäßersatz zur Herstellung von künstlichen Blutgefäßen verwendet. Unter Einfluss des magnetischen Wechselfeldes erfolgt als Formänderung eine Expansion der künstlichen Gefäßwände in Längsrichtung. FIG. 9a) zeigt schematisch diesen Übergang. Vorteilhafterweise kann dadurch ein kurzes Gefäßersatzstück unter minimaler Verletzung des natürlichen Gefäßes eingesetzt und anschließend auf die gewünschte Länge expandiert werden. Somit erfolgt dann eine Stabilisierung der Gefäßwand über die Operationsstelle hinaus, was für den Heilungsprozess vorteilhaft ist. In FIG. 9b) ist die für das künstliche Blutgefäß verwendete Porenstruktur gezeigt. Der das künstliche Blutgefäß bildende Kanal ist in schwarz zu erkennen.

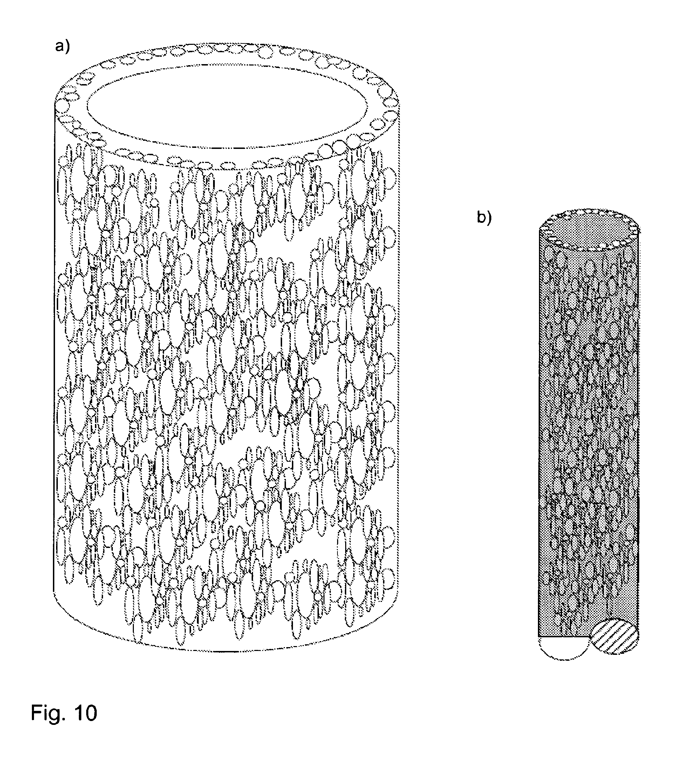

FIG. 10 zeigt schematisch die Verschäumung verschiedener geometrischer Körper für den medizinischen Gebrauch. Eine röhrenförmige Geometrie, wie sie bei Kathetern und Gefäßersatz zum Einsatz kommt, wird auf der Mantelfläche selektiv verschäumt (FIG. 10 a)). Massive zylindrische Körper finden beispielsweise in Form von Monofilamenten als Nahtmaterial Verwendung. FIG.10b) zeigt ein solches verschäumtes Monofilament schematisch.

Bezugszeichenliste

10   Glasgefäß
12   destilliertes Wasser
14   Kunststoffunterlage
16   Spulen eines Induktors
18   Probe
20   Schaumhülle
22   Thermosensoren

**Patentansprüche**

**1.** Artikel mit mindestens einem Schaltabschnitt, wobei der mindestens eine Schaltabschnitt umfasst

a) einen Formgedächtniscompound, der mindestens ein thermo-programmierbares Formgedächtnispolymer und darin eingearbeitete Partikel enthält, die geeignet sind, sich in einem alternierenden elektromagnetischen Feld zu erwärmen, wobei der Formgedächtniscompound eine Wärmeübergangszahl $h_{schalt}$ aufweist;
b) einen Isolierbereich, der den Formgedächtniscompound a) umgibt und der eine Wärmeübergangszahl $h_{iso}$

aufweist;

wobei $h_{iso} < h_{schalt}$ ist.

**2.** Artikel nach Anspruch 1,
**dadurch gekennzeichnet, dass**
das Verhältnis $h_{iso} / h_{schalt} \leq 0{,}9$ ist, bevorzugt $\leq 0{,}5$, besonders bevorzugt $\leq 0{,}3$, ganz besonders bevorzugt $\leq 0{,}1$.

**3.** Artikel nach einem der vorhergehenden Ansprüche,
**dadurch gekennzeichnet, dass**
der Isolierbereich im Wesentlichen keine Partikel aufweist, die geeignet sind, sich in einem alternierenden elektromagnetischen Feld zu erwärmen.

**4.** Artikel nach einem der vorhergehenden Ansprüche,
**dadurch gekennzeichnet, dass**
der Isolierbereich ein Material aufweist oder aus diesem besteht, dessen spezifische Wärmeleitfähigkeit $_{iso}$ geringer ist als die spezifische Wärmeleitfähigkeit $_{schalt}$ des mindestens einen thermo-programmierbaren Formgedächtnispolymers des Formgedächtniscompounds.

**5.** Artikel nach einem der vorhergehenden Ansprüche,
**dadurch gekennzeichnet, dass**
der Isolierbereich eine Strukturierung aufweist, die sich von der Strukturierung des Formgedächtniscompounds unterscheidet.

**6.** Artikel nach einem der vorhergehenden Ansprüche,
**dadurch gekennzeichnet, dass**
der Isolierbereich eine Schaumstruktur aufweist, insbesondere eine vorwiegend geschlossenzellige Schaumstruktur.

**7.** Artikel nach einem der Ansprüche 4, 5 oder 6,
**dadurch gekennzeichnet, dass**
das Material des Isolierbereichs ein Gas umfasst, bevorzugt ein Gas, das sich inert Verhält gegenüber dem mindestens einen thermo-programmierbaren Formgedächtnispolymers des Formgedächtniscompounds, besonders bevorzugt $CO_2$ oder Luft ist.

**8.** Artikel nach einem der vorhergehenden Ansprüche,
**dadurch gekennzeichnet, dass**
das Verhältnis des gemittelten Durchmessers des Isolierbereichs und des Formgedächtniscompounds ($D_{iso+schalt}$) zu dem gemittelten Durchmesser des Formgedächtniscompounds ($D_{schalt}$) im Querschnitt des mindestens einen Schaltabschnitts $D_{iso+schalt}/ D_{schalt}$ so gewählt ist, dass im Formgedächtniscompound durch Anregung mit einem alternierenden elektromagnetischen Feld eine Schalttemperatur des mindestens einen Formgedächtnispolymers des Formgedächtniscompounds erreichbar ist, wenn sich der mindestens eine Schaltabschnitt in einer Umgebung mit einer Wärmeübergangzahl befindet, die größer ist, als die Wärmeübergangzahl des Formgedächtniscompounds des Schaltabschnitts.

**9.** Artikel nach Anspruch 8,
**dadurch gekennzeichnet, dass**
das Durchmesserverhältnis $D_{iso+schalt} / D_{schalt}$ so gewählt ist, dass der mindestens eine Schaltabschnitt in einer Umgebung aus Wasser und/oder in einem alternierenden elektromagnetischen Feld mit einer Frequenz von 245 bis 265 kHz und einer Feldstärke von 10 bis 16 kA/m die Schalttemperatur erreicht.

**10.** Artikel nach einem der vorhergehenden Ansprüche,
**dadurch gekennzeichnet, dass**
das Verhältnis des gemittelten Durchmessers des Isolierbereichs und des Formgedächtniscompounds zu dem gemittelten Durchmesser des Formgedächtniscompounds im Querschnitt des mindestens einen Schaltabschnitts $D_{iso+schalt} / D_{schalt} \geq 1{,}01$ ist, bevorzugt $\geq 1{,}05$, besonders bevorzugt $\geq 1{,}1$.

**11.** Artikel nach einem der vorhergehenden Ansprüche,
**dadurch gekennzeichnet, dass**

der Isolierbereich Poren aufweist, die entlang eines Porengrößengradienten über den Querschnitt des Isolierbereichs verteilt vorliegen, wobei die Poren in einem äußeren Bereich des Isolierbereichs im Durchschnitt kleiner sind als die Poren in einem inneren Bereich des Isolierbereichs.

**12.** Artikel nach einem der Ansprüche 1 bis 11 für Anwendungen bei denen der Artikel in Kontakt kommt mit Materialien mit einer Wärmeübergangszahl die größer ist als $h_{schalt}$, insbesondere mit flüssigen Materialien oder Geweben des menschlichen oder tierischen Körpers.

**13.** Artikel nach einem der Ansprüche 1 bis 11 zur Anwendung in einem Verfahren zur chirurgischen oder therapeutischen Behandlung des menschlichen oder tierischen Körpers oder in einem Diagnostizierverfahren, wobei der Artikel in Kontakt gebracht wird mit Bestandteilen eines menschlichen oder tierischen Körpers.

**14.** Verfahren zur Herstellung eines Artikels mit mindestens einem Schaltabschnitt nach einem der Ansprüche 1 bis 11, umfassend die Schritte:

a) Darstellung eines Formgedächtniscompounds des mindestens einen Schaltabschnitts, der mindestens ein thermo-programmierbares Formgedächtnispolymer und darin eingearbeitete Partikel enthält, die geeignet sind, sich in einem alternierenden elektromagnetischen Feld zu erwärmen, wobei der Formgedächtniscompound eine Wärmeübergangszahl $h_{schalt}$ aufweist; und

b) Aufbringen eines Isolierbereichs, der eine Wärmeübergangszahl $h_{iso} < h_{schalt}$ aufweist, auf den Formgedächtniscompound des mindestens einen Schaltabschnitts, sodass der Isolierbereich den Formgedächtniscompound umgibt und

wobei Schritt b) gleichzeitig mit Schritt a) oder nachgelagert zu Schritt a) ausgeführt wird.

**15.** Verfahren nach Anspruch 14,
**dadurch gekennzeichnet, dass**
der Formgedächtniscompound des mindestens einen Schaltabschnitts durch Verformung aus einer Polymerschmelze, durch kontinuierliches und/oder diskontinuierliches, ein- und/oder mehrschichtiges Extrusions- oder Spritzgussverfahren und/oder durch einen Fügeprozess mehrerer vorgefertigter Elemente hergestellt wird.

**16.** Verfahren nach einem der Ansprüche 14 oder 15,
**dadurch gekennzeichnet, dass**
der Isolierbereich durch Verschäumen, durch Sprühen und/oder durch Phaseninversion aufgebracht wird.

**17.** Verfahren nach einem der Ansprüche 14 bis 16,
**dadurch gekennzeichnet, dass**
der Isolierbereich durch Verschäumen und/oder Phaseninversion des mindestens einen thermo-programmierbares Formgedächtnispolymers des Formgedächtniscompounds erzeugt wird.

**18.** Verfahren nach einem der Ansprüche 14 bis 17,
**dadurch gekennzeichnet, dass**
der Isolierbereich durch Verschäumen und/oder Phaseninversion eines oder mehrerer Materialien erzeugt wird, die sich vom mindestens einen thermo-programmierbaren Formgedächtnispolymer des Formgedächtniscompounds unterscheiden.

**Claims**

**1.** An item with at least one switching section, wherein the at least one switching section comprises

a) a shape memory compound containing at least one thermo-programmable shape memory polymer and particles incorporated therein adapted to heat up in an alternating electromagnetic field, wherein the shape memory compound has a heat transfer coefficient $h_{schalt}$;
b) an insulating region surrounding the shape memory compound a) and having a heat transfer coefficient $h_{iso}$;
wherein $h_{iso} < h_{schalt}$.

**2.** The item according to claim 1,
**characterised in that**

the ratio $h_{iso}/h_{schalt}$ is $\leq 0.9$, preferably $\leq 0.5$, particularly preferably $\leq 0.3$, most preferably $\leq 0.1$.

3. The item according to any one of the preceding claims,
   **characterised in that**
   the insulating region has substantially no particles adapted to heat up in an alternating electromagnetic field.

4. The item according to any one of the preceding claims,
   **characterised in that**
   the insulating region has or consists of a material whose specific thermal conductivity $_{iso}$ is smaller than the specific thermal conductivity $_{schalt}$ of the at least one thermo-programmable shape memory polymer of the shape memory compound.

5. The item according to any one of the preceding claims,
   **characterised in that**
   the insulating region has a structuring which is different from the structuring of the shape memory compound.

6. The item according to any one of the preceding claims,
   **characterised in that**
   the insulating region has a foam structure, in particular a predominantly closed-cell foam structure.

7. The item according to any one of claims 4, 5 or 6,
   **characterised in that**
   the material of the insulating region comprises a gas, preferably a gas which is inert to the at least one thermo-programmable shape memory polymer of the shape memory compound, particularly preferably $CO_2$ or air.

8. The item according to any one of the preceding claims,
   **characterised in that**
   the ratio between the mean diameter of the insulating region and the shape memory compound ($D_{iso+schalt}$) and the mean diameter of the shape memory compound ($D_{schalt}$) in the cross-section of the at least one switching section $D_{iso+schalt}/D_{schalt}$ is selected such that a switching temperature of the at least one shape memory polymer of the shape memory compound can be achieved in the shape memory compound by excitation with an alternating electromagnetic field if the at least one switching section is in an environment with a heat transfer coefficient which is higher than the heat transfer coefficient of the shape memory compound of the switching section.

9. The item according to claim 8,
   **characterised in that**
   the diameter ratio $D_{iso+schalt}/D_{schalt}$ is selected such that the at least one switching section reaches the switching temperature in an environment of water and/or in an alternating electromagnetic field with a frequency of 245 to 265 kHz and a field intensity of 10 to 16 kA/m.

10. The item according to any one of the preceding claims,
    **characterised in that**
    the ratio of the mean diameter of the insulating region and the shape memory compound and the mean diameter of the shape memory compound in the cross-section of the at least one switching section $D_{iso+schalt}/D_{schalt}$ is $\geq 1.01$, preferably $\geq 1.05$, particularly preferably $\geq 1.1$.

11. The item according to any one of the preceding claims,
    **characterised in that**
    the insulating region has pores which are present distributed across the cross-section of the insulating region along a pore size gradient, wherein the pores in an outer region of the insulating region are on average smaller than the pores in an inner region of the insulating region.

12. The item according to any one of claims 1 to 11 for applications wherein the article comes into contact with materials having a heat transfer coefficient higher than $h_{schalt}$, in particular with liquid materials or tissues of the human or animal body.

13. The article according to any one of claims 1 to 11 for application in a method for the surgical or therapeutic treatment of the human or animal body or in a diagnosing method, wherein the item is brought into contact with parts of a human or animal body.

14. A method for manufacturing an item with at least one switching section according to any one of claims 1 to 11, comprising the steps of:

   a) preparation of a shape memory compound of the at least one switching section, containing at least one thermo-programmable shape memory polymer and particles incorporated therein adapted to heat up in an alternating electromagnetic field, wherein the shape memory compound has a heat transfer coefficient $h_{schalt}$; and
   b) application of an insulating region having a heat transfer coefficient $h_{iso} < h_{schalt}$ to the shape memory compound of the at least one switching section such that the insulating region surrounds the shape memory compound and
   wherein step b) is carried out simultaneously with step a) or downstream of step a).

15. The method according to claim 14,
   **characterised in that**
   the shape memory compound of the at least one switching section is manufactured from a polymer melt by deformation, by a continuous and/or discontinuous, single layer and/or multilayer extrusion or injection moulding method and/or by a process of joining multiple prefabricated elements.

16. The method according to any one of claims 14 or 15,
   **characterised in that**
   the insulating region is applied by foaming, spraying and/or by phase inversion.

17. The method according to any one of claims 14 or 16,
   **characterised in that**
   the insulating region is generated by foaming and/or phase inversion of the at least one thermo-programmable shape memory polymer of the shape memory compound.

18. The method according to any one of claims 14 or 17,
   **characterised in that**
   the insulating region is generated by foaming and/or phase inversion of one or more materials which differ from the at least one thermo-programmable shape memory polymer of the shape memory compound.

**Revendications**

1. Article ayant au moins une partie de commutation, la partie de commutation au moins au nombre de un comprenant

   a) une composition à mémoire de forme qui contient au moins un polymère à mémoire de forme thermoprogrammable et des particules qui y sont intégrées et qui sont appropriées pour se réchauffer dans un champ électromagnétique alternatif, la composition à mémoire de forme présentant un coefficient de transfert thermique $h_{schalt}$;
   b) une zone isolante qui entoure la composition à mémoire de forme a) et qui présente un coefficient de transfert thermique $h_{iso}$ ;
   avec $h_{iso} < h_{schalt}$.

2. Article selon la revendication 1,
   **caractérisé en ce que**
   le rapport $h_{iso} / h_{schalt}$ est $\leq 0,9$, de préférence $\leq 0,5$, de façon particulièrement préférée $\leq 0,3$, de façon tout à fait préférée $\leq 0,1$.

3. Article selon une des revendications précédentes,
   **caractérisé en ce que**
   la zone isolante ne présente essentiellement pas de particules qui sont appropriées pour se réchauffer dans un champ électromagnétique alternatif.

4. Article selon une des revendications précédentes,
   **caractérisé en ce que**

la zone isolante présente un matériau ou se compose de celui-ci, dont la conductivité thermique $_{iso}$ est plus faible que la conductivité thermique $_{schalt}$ du polymère à mémoire de forme thermoprogrammable au moins au nombre de un de la composition à mémoire de forme.

5. Article selon une des revendications précédentes,
   **caractérisé en ce que**
   la zone isolante présente une structuration qui se distingue de la structuration de la composition à mémoire de forme.

6. Article selon une des revendications précédentes,
   **caractérisé en ce que**
   la zone isolante présente une structure alvéolaire, en particulier une structure alvéolaire principalement à cellules fermées.

7. Article selon une des revendications 4, 5 ou 6,
   **caractérisé en ce que**
   le matériau de la zone isolante comprend un gaz, de préférence un gaz qui se comporte de façon inerte par rapport au polymère à mémoire de forme thermoprogrammable au moins au nombre de un de la composition à mémoire de forme, qui est de façon particulièrement préférée du $CO_2$ ou de l'air.

8. Article selon une des revendications précédentes,
   **caractérisé en ce que**
   le rapport du diamètre moyen de la zone isolante et de la composition à mémoire de forme ($D_{iso+schalt}$) au diamètre moyen de la composition à mémoire de forme ($D_{schalt}$) dans la section transversale de la partie de commutation $D_{iso+schalt}$ / $D_{schalt}$ au moins au nombre de un est choisi de telle sorte que, dans la composition à mémoire de forme, par l'excitation avec un champ électromagnétique alternatif, il est possible d'atteindre une température de commutation du polymère à mémoire de forme au moins au nombre de un de la composition à mémoire de forme si la partie de commutation au moins au nombre de un se trouve dans un environnement avec un coefficient de transfert thermique qui est supérieur au coefficient de transfert thermique de la composition à mémoire de forme de la partie de commutation.

9. Article selon la revendication 8,
   **caractérisé en ce que**
   le rapport de diamètre $D_{iso+schalt}$ / $D_{schalt}$ est choisi de telle sorte que la partie de commutation au moins au nombre de un atteint la température de commutation dans un environnement composé d'eau et/ou dans un champ électromagnétique alternatif d'une fréquence de 245 à 265 kHz et d'une intensité de champ de 10 à 16 kA/m.

10. Article selon une des revendications précédentes,
    **caractérisé en ce que**
    le rapport du diamètre moyen de la zone isolante et de la composition à mémoire de forme au diamètre moyen de la composition à mémoire de forme dans la section transversale de la partie de commutation $D_{iso+schalt}$ / $D_{schalt}$ au moins au nombre de un est $\geq 1,01$, de préférence $\geq 1,05$, de façon particulièrement préférée $\geq 1,1$.

11. Article selon une des revendications précédentes,
    **caractérisé en ce que**
    la zone isolante présente des pores qui sont répartis le long d'un gradient de grandeur de pores sur la section transversale de la zone isolante, les pores étant, dans une zone extérieure de la zone isolante, en moyenne plus petits que les pores dans une zone intérieure de la zone isolante.

12. Article selon une des revendications 1 à 11, pour des applications dans lesquelles l'article vient en contact avec des matériaux ayant un coefficient de transfert thermique qui est supérieur à $h_{schalt}$, en particulier avec des matériaux liquides ou des tissus du corps humain ou du corps animal.

13. Article selon une des revendications 1 à 11, pour une utilisation dans un procédé de traitement chirurgical ou thérapeutique du corps humain ou du corps animal ou dans un procédé de diagnostic, l'article étant mis en contact avec des parties constitutives d'un corps humain ou animal.

14. Procédé de fabrication d'un article ayant au moins une partie de commutation selon une des revendications 1 à 11, comprenant les étapes suivantes :

a) représentation d'une composition à mémoire de forme de la partie de commutation au moins au nombre de un qui contient au moins un polymère à mémoire de forme thermoprogrammable et des particules qui y sont intégrées et qui sont appropriées pour se réchauffer dans un champ électromagnétique alternatif, la composition à mémoire de forme présentant un coefficient de transfert thermique $h_{schalt}$; et

b) application d'une zone isolante, qui a un coefficient de transfert thermique $h_{iso} < h_{schalt}$, sur la composition à mémoire de forme de la partie de commutation au moins au nombre de un de telle sorte que la zone isolante entoure la composition à mémoire de forme, et

l'étape b) étant effectuée en même que l'étape a) ou après l'étape a).

**15.** Procédé selon la revendication 14,
**caractérisé en ce que**
la composition à mémoire de forme de la partie de commutation au moins au nombre de un est fabriquée par formage à partir d'une masse fondue de polymère, par procédé d'extrusion ou de moulage par injection continu et/ou discontinu, à une et/ou à plusieurs couches, et/ou par un processus d'assemblage de plusieurs éléments préfabriqués.

**16.** Procédé selon une des revendications 14 ou 15,
**caractérisé en ce que**
la zone isolante est mise en place par moussage, par aspersion et/ou par inversion de phase.

**17.** Procédé selon une des revendications 14 à 16,
**caractérisé en ce que**
la zone isolante est produite par moussage et/ou inversion de phase du polymère à mémoire de forme thermoprogrammable au moins au nombre de un de la composition à mémoire de forme.

**18.** Procédé selon une des revendications 14 à 17,
**caractérisé en ce que**
la zone isolante est produite par moussage et/ou inversion de phase d'un ou de plusieurs matériaux qui se distinguent du polymère à mémoire de forme thermoprogrammable au moins au nombre de un de la composition à mémoire de forme.

Fig. 1

Fig. 2

Fig. 3

a) 

| Accelerating Voltage 5 kV | Magnification 9x | Detector SE2 | 2 mm |

b)

| Accelerating Voltage 10 kV | Magnification 50000x | Detector SE2 | 0,5 µm |

FIG. 4

FIG. 5

FIG. 6

Fig. 7

Fig. 8

a)

Magnetisches ⟹ Wechselfeld

b)

| Accelerating Voltage 10 kV | Magnification 500x | Detector RBSD | 50 µm |
|---|---|---|---|

Fig. 9

a)

b)

Fig. 10

# IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004056156 A
- WO 2002012409 A
- US 2004219130 A
- US 20050212630 A
- DE 102007061342

**In der Beschreibung aufgeführte Nicht-Patentliteratur**

- **LI F. et al.** Polyurethane / Conducting Carbon Black Composites: Structure, Electric Conductivity, Strain Recovery Behavior, and Their Relationships. *Journal of Applied Polymer Science,* 2000, vol. 75 (1), 68-77
- **GALL K. et al.** Carbon fiber reinforced shape memory polymer composites. *Journal of Inteligent Material Systems and Structures,* 2001, vol. 11 (11), 877-886
- **LIANG C. et al.** Investigation of shape memory polymers and their hybrid composites. *Journal of Inteligent Material Systems and Structures,* 1997, vol. 8 (4), 380-386
- **GALL K. et al.** Shape memory polymer nanocomposites. *Acta Materialia,* 2002, vol. 50, 5115-5126
- **GOTTHARDT R. et al.** Smart materials based on shape memory alloys (SMAs): examples from Europe. *Materials Science Forum 327-328 (Shape Memory Materials),* 2000, 83-90
- **MONKMANN G.J.** Advances in Shape Memory Polymer Actuation. *Mechatronics,* 2000, vol. 10, 489-498